(19) Europäisches Patentamt
European Patent Office
Office européen des brevets

(11) Publication number: 0 084 920 B1

(12) # EUROPEAN PATENT SPECIFICATION

(45) Date of publication of patent specification: 19.04.89

(51) Int. Cl.$^4$: C 07 D 211/22, C 07 D 207/08

(21) Application number: 83200131.7

(22) Date of filing: 05.12.80

(60) Publication number of the earlier application in accordance with Art. 76 EPC: 0 030 526

(54) **New compounds useful as intermediates or starting materials for preparation of therapeutically useful phenyl-azacycloalkanes.**

(30) Priority: 05.12.79 SE 7910026

(43) Date of publication of application: 03.08.83 Bulletin 83/31

(45) Publication of the grant of the patent: 19.04.89 Bulletin 89/16

(84) Designated Contracting States: AT BE CH DE FR GB IT LI LU NL SE

(56) References cited:
US-A-2 975 193
US-A-3 745 171

BULLETIN DE LA SOCIETE CHIMIQUE DE FRANCE, NO. 3, 1968, PARIS (FR), pages 1000-1007. M. JULIA et al.: "Recherches sur les aryl-e pipéridines. II. - Méthoxy phényl (o-, m-, p-), naphtyl et tétralyl-3 pipéridines N-substituées"

(73) Proprietor: Carlsson, Per Arvid Emil
Torild Wulffsgatan 50
S-413 19 Göteborg (SE)

(72) Inventor: Arvidsson, Folke Lars-Erik
Seminariegränd 3
S-752 58 Uppsala (SE)
Inventor: Carlsson, Per Arvid Emil
Torild Wulffsgatan 50
S-413 19 Göteborg (SE)
Inventor: Hacksell, Uli Alf Department of Chemistry
Seeley G Mudd Building 6 Lehigh University
Bethlehem Pennsylvania 18015 (US)
Inventor: Hjorth, John Stephan Mikael
Blavalsgatan 7A
S-414 75 Göteborg (SE)
Inventor: Lindberg, Per Lennart
Knapehall 64
S-436 00 Askim (SE)
Inventor: Nilsson, John Lars Gunnar
Tullinge Strand 30B
S-146 00 Tullinge (SE)
Inventor: Sanchez, Domingo
Snipasägen 24
S-440 03 Floda (SE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 084 920 B1

Courier Press, Leamington Spa, England.

(72) Inventor: **Svensson, Nils Uno Eimer**
**Rallarvägen 6**
**S-740 20 Brunna (SE)**
Inventor: **Wikström, Hakan Vilhelm**
**Gibsons väg 25**
**S-433 61 Partille (SE)**

(74) Representative: **Perry, Robert Edward et al**
**GILL JENNINGS & EVERY 53-64 Chancery Lane**
**London WC2A 1HN (GB)**

## Description

The present invention relates to compounds which are useful as intermediates or starting materials for the preparation of the therapeutically-active 3-phenyl-piperidines described in EP—A—0030526.

Chemical Abstracts *69*: 86776s (1968) discloses compounds of formula IV (see below) in which $R^7$ is $CH_3$ and $Z^3$ is H, $CH_3$, $C_2H_5$, benzyl, phenethyl or 2-(4-nitrophenyl)ethyl. These compounds were prepared for the investigation of pharmalogical properties.

Novel compounds according to the present invention are of formula IV, wherein $Z^3$ is R or $R^6CO$, wherein R is $C_{1-5}$ alkyl, a hydroxyalkyl, dimethylaminoalkyl or methylthioalkyl group having 2—6 carbon atoms in the alkyl part and having the heteroatom bound in a position other than the 1-position, or $C_{3-5}$ alkenyl which is not 1-alkenyl, and $R^6$ is $C_{1-4}$ alkyl, $C_{1-4}$ alkoxy or $C_{2-4}$ alkenyl; and $R^7$ is $C_{1-5}$ alkyl, allyl or benzyl; provided that $Z^3$ is not methyl or ethyl when $R^7$ is $CH_3$; including the acid addition salts thereof.

Preferably, R is alkyl, hydroxyalkyl or alkenyl as defined above, $R^6$ is as defined above, and $R^7$ is $C_{1-5}$ alkyl.

More preferably, $Z^3$ is $C_{3-5}$ alkyl. Most preferably, $R^7$ is $CH_3$ and $Z^3$ is n-propyl, n-butyl, n-pentyl or isopropyl.

Both organic and inorganic acids can be employed to form acid addition salts of the compounds of this invention. Illustrative acids are sulfuric, nitric, phosphoric, hydrochloric, citric, acetic, lactic, tartaric, pamoic, ethanedisulfonic, sulfamic, succinic, cyclohexylsulfamic, fumaric, maleic and benzoic acids. These salts are readily prepared by methods known in the art.

Compounds of the invention are useful in the preparation of N-butyl-3-(3-hydroxyphenyl)piperidine, N-pentyl-3-(3-hydroxyphenyl)piperidine, N-isopropyl-3-(3-hydroxyphenyl)piperidine and, especially, N-n-propyl-3-(3-hydroxyphenyl)piperidine.

The preparation of compounds of the invention will now be described with reference to the accompanying Chart, in which $R^a$ is $C_{1-5}$ alkyl and $R^6$, $R^7$ and $Z^3$ are as defined above. Formulae IVa and IVb are within the scope of formula IV.

The I → III conversion comprises reduction, e.g. with $LiAlH_4$.

The II → III conversion comprises hydrogenation. A compound of formula II in which $R^a$ is other than methyl may be prepared from the corresponding compound in which $R^a$ is methyl by reaction with HBr to give 3-(3-hydroxyphenyl)piperidine which is reacted with the appropriate alkyl halide in the presence of a base.

The III → IVa conversion comprises alkylation with an appropriate alkylating agent. Thus, the starting compound may be treated with an alkyl, hydroxyalkyl, dimethylaminoalkyl, methylthioalkyl, alkenyl or benzyl halide or tosylate RX, wherein X represents Cl, Br, I or p-tosyl, in an organic solvent such as acetonitrile or acetone and in the presence of a base such as $K_2CO_3$ or NaOH. Alternatively, the starting compound may be treated with a carboxylic acid-$NaBH_4$ complex $R—CH_2—COOH—NaBH_4$.

For the formation of a compound of formula IVa wherein R is $CH_3$, which is not obtainable by the last-mentioned reaction, the alkylation reaction may be carried out by treatment with a formaldehyde-$Na(CN)BH_3$ mixture. For the formation of a compound of formula IVa wherein R is hydroxyalkyl, dimethylaminoalkyl or methylthioalkyl, the synthesis may also be carried out by alkylation with an appropriate dihaloalkane giving a monohaloalkyl derivative of IVa, followed by acid or alkaline hydrolysis and reaction with dimethylamine or $CH_3S^-$. Expecially for the formation of a compound of formula IVa wherein R is 2-hydroxyalkyl, the alkylation may also be carried out by reaction with a 1,2-epoxyalkane.

The IVa → V conversion comprises reaction with an acidic nucleophilic reagent such as aqueous HBr or HI, $BHr/CH_3COOH$, $BBr_3$, $AlCl_3$, pyridine-HCl or $(CH_3)SiI$, or with a basic nucleophilic reagent such as $CH_3C_6H_4—S^-$ or $C_2H_5—S^-$.

A compound of formula IV wherein $R^7$ is allyl or benzyl and $Z^3$ = R (but not hydroxyalkyl) is obtained by reacting a corresponding compound of formula V with an allyl or benzyl halide, preferably the chloride or bromide, in the presence of a base such as triethylamine, pyridine or potassium t-butoxide.

The III → IVb conversion comprises N-acylation with an acid chloride of the formula $R^6COCl$ in the presence of a base. The IVb → VI conversion comprises reaction with $BBr_3$.

A compound of formula IV wherein $R^7$ is allyl or benzyl and $Z^3 = COR^6$ is obtained by reacting a corresponding compound of formula VI with an allyl or benzyl halide.

A compound of formula IV in which $R^7$ is allyl or benzyl and $Z^3$ = R may be obtained from the corresponding compound in which $R^3 = COR^6$ by treatment with a reducing agent, preferably a hydride reducing agent such as $LiAlH_4$ or $BH_3$ in an ethereal solvent, or a metal reducing agent such as Na in an alcoholic solvent such as n-butanol.

The following Examples will further illustrate the invention. The preparations illustrate starting materials and intermediates.

### Example 1

N-Butyl-3-(3-methoxyphenyl)piperidine hydrochloride

Butyryl chloride (2.0 g, 0.019 mol) in dry toluene (5 ml) was slowly added to a solution of 3-methoxyphenylpiperidine (2.45 g, 0.013 mol) and triethylamine (1.92 g, 0.013 mol) in dry toluene at 5°C. The mixture was stirred at room temperature for 30 min., whereupon the triethylammonium chloride formed was

filtered off and the solvent evaporated. The crude N-butyryl-3-(3-methoxypheny)piperidine (2.82 g) dissolved in dry tetrahydrofuran (30 ml) was added to a suspension of $LiAlH_4$ (2.0 g) in dry tetrahydrofuran (30 ml) under nitrogen. After reflux for 3 h the mixture was hydrolysed, the precipitate filtered off and the solvent evaporated. The residue dissolved in light petroleum was passed through an alumina column. (Alternatively the residue could be distilled in vacuo). The product was precipitated as the hydrochloride and recrystallized from ethanol/ether yielding the pure product (3.6 g, 88%) mp 130—1°C. Elemental analysis was satisfactory.

Example 2

N-Pentyl-3-methoxyphenyl)piperidine

To a solution of 3-(3-methoxyphenyl)piperidine (3.92 g, 0.02 mol) in $CH_3CN$ (100 ml), solid $K_2CO_3$ (5 g) was added and the mixture was refluxed. A solution of pentyl-iodide (4.5 g, 0.021 mol) in $CH_3CN$ (10 ml) was added dropwise under 30 min. and then the mixture was refluxed for an additional 30 min. The solid was filtered off from the cooled mixture, and the solvent evaporated giving an oil which was chromatographed on a silica gel column with methanol as eluant. Yield 1.3 g (25%) of pure N-pentyl-3-(3-methoxyphenyl)piperidine as an oil. NMR spectrum: δ ($CDCl_3$) 0.7—3.2 (20H, m), 3.75 (3H, s), 6.6—7.0 (3H, m), 7.0—7.4 (1H, m). Elemental analysis was satisfactory.

Example 3

N-Propyl-3-(3-methoxyphenyl)piperidine hydrochloride

$NaBH_4$ (6.08 g, 0.16 mol) was added portionwise under stirring to a solution of propionic acid (38 g, 0.51 mol) in dry benzene (150 ml). The temperature was kept below 15°C for 2 h and then 3-(3-methoxyphenyl)piperidine (6.1 g, 0.032 mol) dissolved in dry benzene (100 ml) was added and the mixture was refluxed for 3 h. The reaction mixture was allowed to reach room temperature and was then extracted with 2.5 M NaOH (200 ml). The aqueous phase was extracted with benzene, all the benzene phases mixed, dried ($Na_2SO_4$) and the solvent evaporated giving an oily residue (6.6 g). The product was precipitated as hydrochloride and recrystallized from methanol/isopropyl ether yielding the pure product (6.2 g, 72%), mp. 191°C. Elemental analysis was satisfactory.

Example 4

N-Propyl-3-(3-methoxyphenyl)piperidine hydrobromide

3-(3-Pyridinyl)methoxybenzene (3.0 g, 0.016 mol) and propyl bromide (2.0 g) were dissolved in dry acetone (50 ml) and allowed to react at 110°C in a high pressure steel vessel. After 20 h the reaction was interrupted and the solvent was evaporated. The residual quaternary N-propyl-3-(3-methoxyphenyl)-pyridinium bromide was hydrogenated ($PtO_2$) in methanol at r.t. and 1.01 kPa (760 mm Hg). The $H_2$-uptake ceased after 24 h. The catalyst was filtered off and the solvent evaporated. The hydrobromide was recrystallized from ethanol/ether giving 2.63 g (70%) of the pure product, mp. 155—156°C. Elemental analysis was satisfactory.

Preparation 1

3-(3-Pyridinyl)methoxybenzene

This substance was prpared by a dichlorobis(triphenylphosphine)nickel (II) catalyzed reaction between 3-methoxyphenylmagnesium bromide (from 50 g of 3-bromoanisole and 5.9 g of Mg in THF) and 31.8 g of 3-bromopyridine. Yield 23.1 g (62%), bp. 102°C/0.21 Pa (0.15 mmHg), mp. (HCl) 187.5—9°C.

Preparation 2

3-(3-Methoxyphenyl)piperidine hydrochloride

To a solution of 3-(33-pyridinyl)methoxybenzene (22.0 g, 0.099 mol) in methanol (250 ml), $PtO_2$ (2 g) and conc. HCl (30 ml) were added and the mixture was hydrogenated at 0.34 MPa in a Parr apparatus. After complete hydrogenation, the catalyst was filtered off. Most of the solvent was evaporated; the residue was made alkaline with 1M NaOH and extracted with ether. The ether phase was dried ($Na_2SO_4$) and the solvent evaporated, giving 18 g of the amine product. The hydrochloride was made and then recrystallized from ethanol/ether, yielding 20.9 g (93%), mp. 137—138.5°C. Elemental analysis was satisfactory.

Preparation 3

N-propyl-3-(3-hydroxyphenyl)piperidine hydrobromide

N-Propyl-3-(3-methoxyphenyl)piperidine hydrochloride (7.0 g, 0.026 mol) was suspended in 48% HBr (200 ml). The mixture was refluxed under nitrogen for 3 h. The hydrobromic acid was evaporated and the residue was recrystallized from ethanol/ether, yielding the pure product (6.7 g, 86%) mp. 146—7.5°C. Elemental analysis was satisfactory.

Example 5

N-Propyl-3-(3-hydroxyphenyl)piperidine (0.5 g, 0.0023 mol) was dissolved in $CH_2Cl_2$ (50 ml). Triethylamine (1 ml) and benzoyl chloride (0.5 ml, 0.004 mol) were added and the mixture was stirred at r.t. for 48 h. The solvent was evaporated and the residue was partitioned between ether and water. The ether

phase was dried ($Na_2SO_{44}$) and the solvent evaporated, giving an oily residue which was eluted through a short silica gel column with methanol as eluant. Evaporation of the solvent gave an oily residue (300 mg). The oil was dissolved in ether and HCl-saturated ether was added. Filtration and drying gave the desired compound in a crystalline form. Yield 13%, mp. 170°C.

Example 6

N-Propyl-3-(3-allyloxyphenyl)piperidine hydrochloride

A solution of N-propionyl-3-(3-allyloxyphenyl)piperidine (0.35 g, 0.0013 mol) in dried ether (25 ml) was added dropwise to a suspension of $LiAlH_4$ (0.35 g) in dried ether under nitrogen with stirring, and the mixture was refluxed for 30 min. $H_2O$ (0.35 ml), 15% NaOH (0.35 ml) and $H_2O$ (1 ml) were added and the precipitated crystals were filtered off and washed with ether. The solution was dried with $Na_2SO_4$. Evaporation to dryness gave an oily residue which was dissolved in ether. Addition of HCl-saturated ether resulted in precipitation of white crystals. The crystals were centrifugated and dried. Yield 0.185 g (49%). NMR spectrum: δ ($CDCl_3$) 0.92 (3H, t), 1.2—3.2 (16H, m), 4.45—4.65 (2H, m), 5.15—5.30 (1H, m), 5.30—5.60 (2H, m), 5.80—6.40 (1H, m), 6.55—6.90 (3H, m), 7.00—7.35 (1H, m).

Example 7

N-Propyl-3-(3-benzyloxyphenyl)piperidine hydrochloride

A mixture of N-propyl-3-(3-hydroxyphenyl)piperidine hydrobromide (1.0 g, 0.0033 mol), potassium t-butoxide (1.0 g, 0.009 mol) and benzyl chloride (1.0 g, 0.009 mol) in t-butanol (25 ml) was refluxed for 1 h. Water was added and the mixture extracted with ether. The organic phase was dried with $Na_2SO_4$ and evaporated to dryness giving a pale yellow oily residue. The residue was chromatographed through a silica gel column with methanol as eluant. The pertinent fractions were collected and evaporated to dryness. The oily residue was dissolved in ether and HCl-saturated ether was added, giving white crystals. Evaporation and treatment of the residue with acetone gave 0.60 g (52%) of the desired product as white crystals, mp. 171°C.

Examples 8 to 15

According to the method of Example 1 or Example 2, further compounds of formula IV were prepared and recrystallized as acid addition salts from ethanol/ether or isolated as the bases. Data are given in the following Table.

| Ex. | $R^7$ | $Z^3$ | Salt/ base | Method of Ex. No. | Data | Yield[a] (%) |
|---|---|---|---|---|---|---|
| 8 | $CH_3$ | $—CH(CH_3)_2$ | base | 2 | NMR[b] | 43 |
| 9 | $CH_3$ | $—CH_2C(CH_3)_3$ | base | 1 | NMR[c] | 81 |
| 10 | $CH_3$ | $—CH_2CH{=}CHH_2$ | base | 2 | NMR[d] | 30 |
| 11 | $CH_3$ | $—CH_2CH_2N(CH_3)_2$ | 2.HCl | 2 | mp. 165—170°C dec. | 64[e] |
| 12 | $CH_3$ | $—COC_2H_5$ | — | 1 | IR[f] | — |
| 13 | $CH_3$ | $—COC_3H_7$ | — | 1 | IR[g] | — |
| 14 | $CH_3$ | $—COC(CH_3)_3$ | — | 1 | NMR, IR[h] | 91 |
| 15 | $CH_3$ | $—CH_2CH_2CH_2OH$ | | 1[i] | | |

a) Calculated on the starting phenylpiperidine or pyridinylbenzene.
b) δ ($CDCl_3$) 1.0 (6H, d), 1.0—3.1 (10H, m), 3.7 (3H, s), 6.55—6.95 (3H, m), 6.95—7.35 (1H, m).
c) δ ($CDCl_3$) 1.2 (9H, s), 1.2—3.1 (11H, m), 3.7 (3H, s), 6.6—6.9 (3H, m), 6.9—7.35 (1H, m).
d) δ ($CDCl_3$) 1.3—3.3 (11H, m), 3.8 (3H, s), 4.9—5.4 (2H, m), 5.55—6.3 (1H, m), 6.6—7.0 (3H, m), 7.0—7.4 (1H, m).
e) N,N-dimethyl-2-chloroethylamine hydrochloride used as alkylating agent. The product was submitted for elemental analysis (C, H, N); the analysis was satisfactory.
f) νmax 1640 (C=O), 1250 ($ArOCH_3$).
g) νmax 1638 (C=O), 1255 ($ArOCH_3$).
h) δ ($CDCl_3$) 1.3 (9H, s), 1.4—3.0 (9H, m), 3.8 (3H, s), 6.65—6.95 (3H, m), 7.1—7.45 (1H, m); νmax 1650 $cm^{-1}$.
i) Method modified by using ethyl 3-bromopropionate as alkylating agent, and effecting reduction of the intermediate ester.

CHART

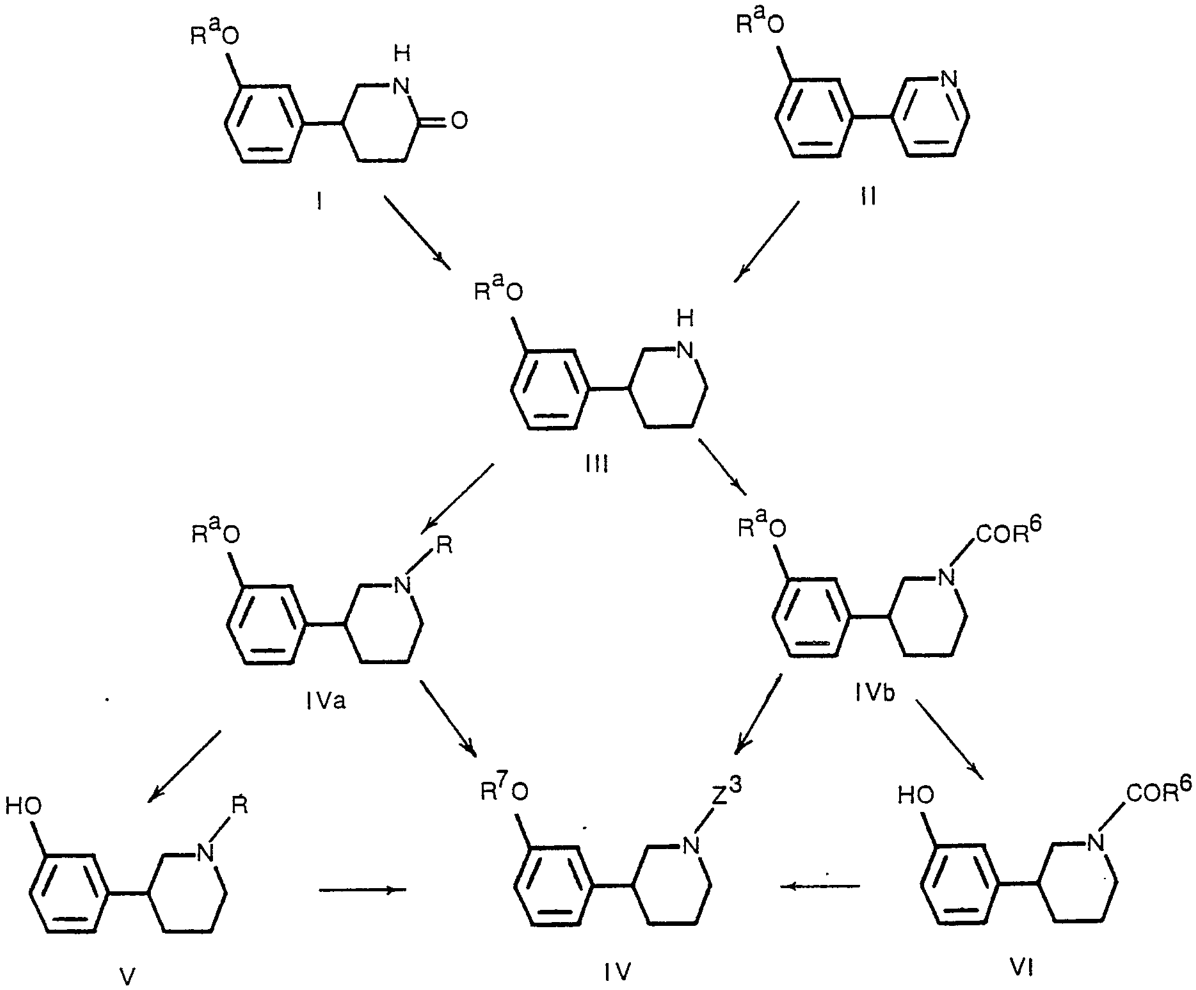

I II III IVa IVb V IV VI

**Claims for the Contracting States: BE CH DE FR GB IT LI LU NL SE**

1. A compound of the formula

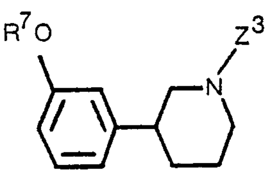

IV

wherein $Z^3$ is R or $R^6CO$, wherein R is $C_{1-5}$ alkyl, a hydroxyalkyl, dimethylaminoalkyl or methylthioalkyl group having 2—6 carbon atoms in the alkyl part and having the heteroatom bound in a position other than the 1-position, or $C_{3-5}$ alkenyl which is not 1-alkenyl, and $R^6$ is $C_{1-4}$ alkyl, $C_{1-4}$ alkoxy or $C_{2-4}$ alkenyl; and $R^7$ is $C_{1-5}$ alkyl, allyl or benzyl; provided that $Z^3$ is not methyl or ethyl when $R^7$ is $CH_3$; or an acid addition salt thereof.

2. A compound according to claim 1, wherein R is alkyl, hydroxyalkyl or alkenyl as defined in claim 1, $R^6$ is as defined in claim 1, and $R^7$ is $C_{1-5}$ alkyl.

3. A compound according to claim 1 or claim 2, wherein $Z^3$ is $C_{3-5}$ alkyl.

4. A compound according to claim 1, wherein $R^7$ is $CH_3$ and $Z^3$ is n-propyl, n-butyl, n-pentyl or isopropyl.

**Claims for the Contracting State: AT**

1. A process for preparing a compound of the formula

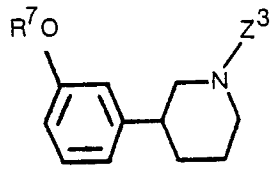

IV

wherein $Z^3$ is R or $R^6CO$, wherein R is $C_{1-5}$ alkyl, a hydroxyalkyl, dimethylaminoalkyl or methylthioalkyl group having 2—6 carbon atoms in the alkyl part and having the heteroatom bound in a position other than the 1-position, or $C_{3-5}$ alkenyl which is not 1-alkenyl, and $R^6$ is $C_{1-4}$ alkyl, $C_{1-4}$ alkoxy or $C_{2-4}$ alkenyl; and $R^7$ is $C_{1-5}$ alkyl, allyl or benzyl; provided that $Z^3$ is not methyl or ethyl when $R^7$ is $CH_3$; or an acid addition salt thereof; which comprises

(i) when $Z_3$ is R and $R_7$ is alkyl, reacting the corresponding compound in which $Z_3$ = H with an alkylating agent of formula RX, wherein X is Cl, Br, I or p-toxyl, in an organic solvent and in the presence of a base, or (when $Z_3$ is not $CH_3$) with a complex of the formula $R—CH_2—COOH—NaBH_4$, or (when $Z_3$ is $CH_3$) with a formaldehyde-$Na(CN)BH_3$ mixture;

(ii) when $Z_3$ is hydroxyalkyl, dimethylaminoalkyl or methylthioalkyl and $R_7$ is alkyl, reacting the corresponding compound wherein $Z_3$ = H with the appropriate dihaloalkane, followed by acid or alkaline hydrolysis and reaction with dimethylamine or $Ca_3S^-$, or (when $Z_3$ is 2-hydroxyalkyl) with a 1,2-epoxyalkane;

(iii) when $Z_3$ is R other than hydroxyalkyl and $R_7$ is allyl or benzyl, reacting a corresponding compound in which $R_7$ = H with an allyl or benzyl halide in the presence of a base;

(iv) when $Z_3$ is $R_6CO$ and $R_7$ is alkyl, reacting a corresponding compound in which Z = H with an acid chloride of the formula $R_6COCl$ in the presence of a base;

(v) when $Z_3$ is $R_6CO$ and $R_7$ is allyl or benzyl, reacting a corresponding compound in which $R_7$ = H with an allyl or benzyl halide; or

(vi) when $Z_3$ is R and $R_7$ is allyl or benzyl, reacting the corresponding compound in which $Z_3$ = $R_6CO$ with a reducing agent.

2. A process according to claim 1, wherein R is alkyl, hydroxyalkyl or alkenyl as defined in claim 1, $R^6$ is as defined in claim 1, and $R^7$ is $C_{1-5}$ alkyl.

3. A process according to claim 1 or claim 2, wherein $Z^3$ is $C_{3-5}$ alkyl.

4. A process according to claim 1, wherein $R^7$ is $CH_3$ and $Z^3$ is n-propyl, n-butyl, n-pentyl or isopropyl.

**Patentansprüche für die Vertragsstaaten: BE CH DE FR GB IT LI LU NL SE**

1. Eine Verbindung der Formel

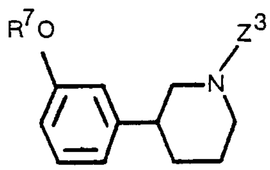

IV

worin $Z^3$ R oder $R^6CO$ bedeutet, worin R $C_{1-5}$ Alkyl, eine Hydroxylalkyl-, Dimethylaminoalkyl- oder Methylthioalkylgruppe mit 2 bis 6 Kohlenstoffatomen in dem Alkylrest und welche an einer anderen Stelle als der 1-Stelle das Heteroatom gebunden trägt, oder $C_{3-5}$ Alkenyl, welches kein 1-Alkenyl darstellt, bedeutet und worin $R_6$ $C_{1-4}$ Alkyl, $C_{1-4}$ Alkoxy oder $C_{2-4}$ Alkenyl bedeutet; und $R^7$ $C_{1-5}$ Alkyl, Allyl oder Benzyl bedeutet; mit dem Proviso, daß $Z^3$ nicht Methyl oder Ethyl bedeutet, wenn $R^7$ $CH_3$ bedeutet; oder ein Säureadditionssalz davon.

2. Verbindung nach Anspruch 1, worin R Alkyl, Hydroxyalkyl oder Alkenyl, wie in Anspruch 1 definiert, bedeutet, $R^6$, wie in Anspruch 1 definiert ist, und $R^7$ $C_{1-5}$ Alkyl bedeutet.

3. Verbindung nach Anspruch 1 oder 2, worin $Z^3$ $C_{3-5}$ Alkyl bedeutet.

4. Verbindung nach Anspruch 1, worin $R^7$ $CH_3$ bedeutet und $Z^3$ n-Propyl, n-Butyl, n-Pentyl oder Isopropyl bedeutet.

**Patentansprüche für den Vertragsstaat: AT**

1. Verfahren zum Herstellen einer Verbindung der Formel

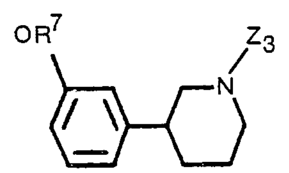

IV

worin $Z_3$ R oder $R_6CO$ bedeutet, worin R $C_{1-5}$ Alkyl, eine Hydroxylalkyl-, Dimethylaminoalkyl- oder Methylthioalkylgruppe mit 2 bis 6 Kohlenstoffatomen in dem Alkylrest und welche an einer anderen Stelle als der 1-Stelle das Heteroatom gebunden trägt, oder $C_{3-5}$ Alkenyl, welches kein 1-Alkenyl darstellt, bedeutet und worin $R_6$ $C_{1-4}$ Alkyl, $C_{1-4}$ Alkoxy oder $C_{2-4}$ Alkenyl bedeutet; und $R_7$ $C_{1-5}$ Alkyl, Allyl oder Benzyl bedeutet; mit dem Proviso, daß $Z_3$ nicht Methyl oder Ethyl bedeutet, wenn $R^7$ $CH_3$ bedeutet; oder ein Säureadditionssalz davon, welches folgende Schritte umfaßt:

(i) wenn $Z_3$ R bedeutet und $R_7$ Alkyl bedeutet, wird die entsprechende Verbindung, in welcher $Z_3$=H mit einem Alkylierungsagens der Formel RX, worin X Cl, Br, I oder p-Tosyl bedeutet, in einem organischen Lösungsmittel in Gegenwart einer Base oder (wenn $Z_3$ nicht $CH_3$ bedeutet) mit einem Komplex der Formel $R—CH_2—COOH—NaBH_4$, oder (wenn $Z_3$ $CH_3$ bedeutet) mit einer Formaldehyd $—Na(CN)BH_3$ Mischung umgesetzt;

(ii) wenn $Z_3$ Hydroxyalkyl, Dimethylaminoalkyl oder Methylthioalkyl bedeutet und $R_7$ Alkyl bedeutet, wird die entsprechende Verbindung, in welcher $Z_3$=H mit dem geeigneten Dihaloalkan, und anschließender saurer oder alkalischer Hydrolyse und Reaktion mit Dimethylamin oder $Ca_3S^-$, oder (wenn $Z_3$ 2-Hydroxyalkyl bedeutet) mit einem 1,2-Epoxyalkan umgesetzt;

(iii) wenn $Z_3$ etwas anderes als Hydroxyalkyl bedeutet und $R_7$ Allyl oder Benzyl bedeutet, wird eine entsprechende Verbindung, in welcher $R_7$=H mit einem Allyl- oder Benzylhalogenid in Gegenwart einer Base umgesetzt;

(iv) wenn $Z_3$ $R_6CO$ bedeutet und $R_7$ Alkyl bedeutet, wird eine entsprechende Verbindung, in welcher Z=H mit einem Säurechlorid der Formel $R_6COCl$ in Gegenwart einer Base umgesetzt;

(v) wenn $Z_3$ $R_6CO$ bedeutet und $R_7$ Allyl oder Benzyl bedeutet, wird eine entsprechende Verbindung, in welcher $R_7$=H mit einem Allyl- oder Benzylhalogenid umgesetzt; oder

(vi) wenn $Z_3$ R bedeutet und $R_7$ Allyl oder Benzyl bedeutet, wird die entsprechende Verbindung, in welcher $Z_3$=$R_6CO$ mit einem Reduktionsmittel umgesetzt.

2. Verfahren nach Anspruch 1, worin R Alkyl, Hydroxyalkyl oder Alkenyl wie in Anspruch 1 definiert bedeutet, $R_6$ wie in Anspruch 1 definiert ist und $R_7$ $C_1$—$C_5$ bedeutet.

3. Verfahren nach Anspruch 1 oder 2, worin $Z_3$ $C_{3-5}$ Alkyl bedeutet.

4. Verfahren nach Anspruch 1, worin $R_7$ $CH_3$ bedeutet und $Z_3$ n-Propyl, n-Butyl, n-Pentyl oder Isopropyl bedeutet.

**Revendications pour les Etats contractants: BE CH DE FR GB IT LI LU NL SE**

1. Composé de formule

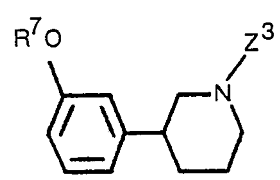

IV

dans laquelle $Z^3$ représente un groupe R ou $R^6CO$, R représente un groupe alkyle en $C_1$ à $C_5$, un groupe hydroxyalkyle, diméthylaminoalkyle ou méthythioalkyle ayant 2 à 6 atomes de carbone dans la portion alkyle et dont l'hétéroatome est fixé en une position autre que la position 1, ou un groupe alcényle en $C_3$ à $C_5$ qui n'est pas un groupe 1-alcényle, et $R^6$ représente un groupe alkyle en $C_1$ à $C_4$, alkoxy en $C_1$ à $C_4$ ou alcényle en $C_2$ à $C_4$; et $R^7$ représente un groupe alkyle en $C_1$ à $C_5$, allyle ou benzyle; sous réserve que $Z^3$ ne représente pas un groupe méthyle ou éthyle lorsque $R^7$ représente un groupe $CH_3$, ou un de ses sels d'addition d'acides.

2. Composé suivant la revendication 1, dans lequel R représente un groupe alkyle, hydroxyalkyle ou alcényle répondant à la définition suivant la revendication 1, $R_6$ est tel que défini dans la revendication 1 et $R^7$ représente un groupe alkyle en $C_1$ à $C_5$.

3. Composé suivant la revendication 1 ou la revendication 2, dans lequel $Z^3$ représente un groupe alkyle en $C_3$ ad $C_5$.

4. Composé suivant la revendication 1, dans lequel $R^7$ représente un groupe $CH_3$ et $Z^3$ représente un groupe n-propyle, n-butyle, n-pentyle ou isopropyle.

**Revendications pour l'Etat contractant: AT**

1. Procédé de préparation d'un composé de formule

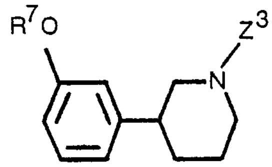

IV

dans laquelle $Z^3$ représente un groupe R ou $R^6CO$, R représente un groupe alkyle en $C_{1-5}$, un groupe hydroxyalkyle, diméthylaminoalkyle ou méthythioalkyle ayant 2 à 6 atomes de carbone dans la portion alkyle et dont l'hétéroatome est fixé en une position autre que la position 1, ou un groupe alcényle en $C_3$ à $C_5$ qui n'est pas un groupe 1-alcényle, et $R^6$ représente un groupe alkyle en $C_1$ à $C_4$, alkoxy en $C_1$ à $C_4$ ou alcényle en $C_2$ à $C_4$; et $R^7$ représente un groupe alkyle en $C_1$ à $C_5$, allyle ou benzyle; sous réserve que $Z^3$ ne représente pas un groupe méthyle ou éthyle lorsque $R^7$ représente un groupe $CH_3$, ou un de ses sels d'addition d'acides; qui consiste

(i) lorsque $Z^3$ représente R et $R^7$ représente un groupe alkyle, à faire réagir le composé correspond dans lequel $Z^3$ est égal à H avec un agent d'alkylation de formule RX, dans laquelle X représente Cl, Br, I ou un groupe p-tosyle, dans un solvant organique et en présence d'une base, ou (lorsque $Z^3$ ne représente pas un group $CH_3$) avec un complexe de formule $R—CH_2—COOH—NaBH_4$, ou bien (lorsque $Z^3$ représente un groupe $CH_3$) avec un mélange de formaldéhyde-$Na(CN)BH_3$;

(ii) lorsque $Z^3$ représente un groupe hydroxyalkyle, diméthylaminoalkyle ou méthylthioalkyle et $R^7$ représente un groupe alkyle, à faire réagir le composé correspondant dans lequel $Z^3$ est égal à H avec le dihalogénoalcane approprié, puis à effectuer une hydrolyse acide ou alcaline et une réaction avec la diméthylamine ou $Ca_3S^-$, ou bien (lorsque $Z^3$ représente un groupe 2-hydroxyalkyle) avec un 1,2-époxyalcane;

(iii) lorsque $Z^3$ représente un groupe R autre qu'un groupe hydroxyalkyle et $R^7$ représente un groupe allyle ou benzyle, à faire réagir un composé correspondant dans lequel $R^7$ est égal à H avec un halogénure d'allyle ou benzyle en présence d'une base;

(iv) lorsque $Z^3$ représente un groupe $R_6CO$ et $R^7$ représente un groupe alkyle, à faire réagir un composé correspondant dans lequel Z est égal à H avec un chlorure d'acide de formule $R_6COCl$ en présence d'une base;

(v) lorsque $Z^3$ représente un groupe $R_6CO$ et $R^7$ représente un groupe allyle ou benzyle, à faire réagir un composé correspondant dans lequel $R^7$ est égal à H avec un halogénure d'allyle ou de benzyle; ou bien

(vi) lorsque $Z^3$ représente un groupe R et $R^7$ représente un groupe allyle ou benzyle, à faire réagir le composé correspondant dans lequel $Z^3$ représente un groupe $R_6CO$ avec un agent réducteur.

2. Procédé suivant la revendication 1, dans lequel R représente un groupe alkyle, hydroxyalkyle ou alcényle répondant à la définition suivant la revendication 1, $R^6$ est tel que défini dans la revendication 1 et $R^7$ représente un groupe alkyle en $C_1$ à $C_5$.

3. Procédé suivant la revendication 1 ou la revendication 2, dans lequel $Z^3$ représente un groupe alkyle en $C_3$ à $C_5$.

4. Procédé suivant la revendication 1, dans lequel $R^7$ représente un groupe $CH_3$ et $Z^3$ représente un groupe n-propyle, n-butyle, n-pentyle ou isopropyle.